# EUROPEAN PATENT APPLICATION

(11) **EP 4 620 451 A1**
(43) Date of publication of application: **24.09.2025**
(21) Application number: 24165495.3
(22) Date of filing: 22.03.2024
(51) Int. Cl.: A61K 6/35

(54) **DENTURE ADHESIVE**

(71) Applicant: Haleon UK IP Limited, Weybridge, Surrey KT13 0NY (GB)
(72) Inventor: The designation of the inventor has not yet been filed
(74) Representative: Haleon Patent Department

(57) **Abstract**

The present invention relates to denture adhesive compositions providing improvements in hold (adhesion over time) whilst having excellent food sealing properties and other characteristics contributing to user experience. The compositions comprise: (a) an alkyl vinyl ether-maleic acid copolymer or salt thereof, (b) carboxymethylcellulose, (c) an oil component and (d) petrolatum.

## Description

### FIELD OF THE INVENTION

The present invention relates to denture adhesive compositions providing improvements in hold (adhesion over time) whilst having excellent food sealing properties and other characteristics contributing to user experience. The compositions comprise: (a) from about 31 to about 34 percent by weight (%w/w) of an alkyl vinyl ether-maleic acid copolymer or salt thereof, (b) from about 15 to about 23 percent by weight (%w/w) of carboxymethylcellulose, (c) from about 16 to about 18 percent by weight (%w/w) of an oil component and (d) from about 20 to about 30 percent by weight (%w/w) of petrolatum.

### BACKGROUND TO THE INVENTION

Full or partial dentures are forms of dental appliance intended to be worn in the mouth as substitutes serving as a replacement for some or all of the teeth usually found in the oral cavity. When using dentures, it is common to use an adhesive to help adhere the dental appliance to a surface of the oral cavity, such as the palate or a gum ridge. Such denture adhesives secure or temporarily fix dentures within the mouth, improving retention of the dentures, whilst also being releasable so that the denture wearer may remove the dentures for cleaning and maintenance. It is also recommended to remove dentures at the end of the day. Denture adhesives can provide an improvement in the denture wearing experience even if the denture fits well. Denture adhesives may also be beneficial in situations where dentures might not fit perfectly, for example as a consequence of natural shrinkage and changes in the gum or oral mucosa over time.

Denture adhesive compositions, (also known as fixative compositions) fill the interstices between the dentures and the gums or tissues. Prior to placement of the denture in the oral cavity, the denture adhesive is applied to the denture plate surface to provide uniform contact with the gums and oral tissues.

Denture adhesive compositions need to fulfill a number of, sometimes competing, criteria in order to function effectively: they should develop a high degree of tack upon contact with saliva so that the dentures are held in place as soon as they are seated in the mouth, possess sufficient cohesive strength to withstand the stresses of mastication which act to dislodge the denture and provide a seal against ingress of food residue beneath the denture. Further, the organoleptic properties of the denture adhesive composition, such as mouthfeel and consistency should be acceptable to the user and the composition should not be too messy or sticky to apply.

Denture adhesives are generally sold as a cream, liner or strip, liquid, gel or powder, and examples are known in the art including commercially available denture adhesives such as POLIGRIP Flavour Free denture adhesive cream (GlaxoSmithKline), POLIGRIP Cushion and Comfort denture adhesive cream (GlaxoSmithKline) and Fixodent PLUS Best Hold Premium denture adhesive cream (P&G). WO/1992/22280 (Richardson Vicks, Inc.) discloses denture stabilizing compositions comprising mixed partial salts of lower alkyl vinyl ether-maleic acid copolymers. WO/2013/054236 (Procter and Gamble) discloses denture adhesives comprising a mixture of a partial salt of a copolymer of methyl vinyl ether-maleic acid, sodium carboxy methyl cellulose and a cohesion builder component in an amount from about 0.001% to about 10% by weight of the denture adhesive composition. WO/2016/091738 (Glaxo Group Ltd) discloses denture adhesive compositions comprising mixed partial salts of lower alkyl vinyl ether-maleic acid copolymers, sodium carboxy methyl cellulose and a bioactive glass. WO/2013/054236 (Procter and Gamble) discloses denture adhesive compositions comprising a cohesion builder component to increase cohesion when hydrated.

Despite considerable effort to develop denture adhesive compositions over the years, much of this work has focused on hold particularly on adhesive strength and longevity. However, many studies point to food entrapment and/or accumulation under dentures as one of the most common complaints, leading to pain and discomfort (see for example, Munoz-Viveros, C., et al. Journal of Dental Research, 2011, vol. 90, no Spec Iss A). Ingress of food particles may also lead to concerns around oral hygiene generally. Thus, there remains a need for further denture adhesives offering improved user experience in other areas such as food seal/blocking, reducing ingress and trapping of food particles in the interstice between a subject's denture and the oral mucosa.

### SUMMARY OF THE INVENTION

The Inventors have developed compositions with desirable adhesive properties, particularly for use with dental appliances such as dentures.

In a First Aspect, there is provided a denture adhesive composition comprising: (a) from about 31 to about 34 percent by weight (%w/w) of an alkyl vinyl ether-maleic acid copolymer or salt thereof, (b) from about 15 to about 23 percent by weight (%w/w) of carboxymethylcellulose, (c) from about 16 to about 18 percent by weight (%w/w) of an oil component and (d) from about 20 to about 30 percent by weight (%w/w) of petrolatum.

Particularly the alkyl vinyl ether-maleic acid copolymer or salt thereof is a poly(methylvinylether/maleic acid) salt. More particularly the alkyl vinyl ether-maleic copolymer or salt thereof is a poly(methylvinylether/maleic acid) Sodium-Calcium Mixed Partial Salt.

Particularly, the carboxymethylcellulose has a viscosity of about 1000 to about 2800 centipoise (cP) (measured in a 1% solution at 25°C). More particularly, the carboxymethylcellulose consists of fine particles of which at least 80% pass through a 200 U.S. mesh sieve (equivalent to a 74 µm mesh sieve). Preferably, the carboxymethylcellulose is sodium carboxymethylcellulose, for example sodium carboxymethylcellulose optionally having a degree of substitution (DS) of from 0.65 to 0.95, particularly about 0.7.

Particularly the oil component is light mineral oil.

Particularly the petrolatum has a cone penetration consistency value of from about 100 to about 200 (mm/10 at 25oC). More particularly the petrolatum has a cone penetration consistency value of from about 130 to about 195 (mm/10 at 25oC).

Particularly, following preparation, the denture adhesive compositions of the Invention have a viscosity of at least 500,000 centipoise (cP). Particularly, following preparation, the denture adhesive compositions of the Invention have a viscosity of no more than 2,500,000 cP, more particularly no more than 2,000,000 cP. Particularly, following preparation, the denture adhesive compositions of the Invention have a viscosity of from 500,000 to 2,500,000 centipoise (cP), more particularly of from 500,000 to 2,000,000 cP. Particularly the compositions have a pH of from 4.0 to 10, more particularly a pH of from 5.0 to 8.0 and still yet more particularly a pH of from 6.5 to 7.5. More particularly the denture adhesive compositions of the Invention have (i) a viscosity of from 500,000 to 2,000,000 centipoise (cP) and/or (ii) a pH of from 4.0 to 10, more particularly a pH of from 5.0 to 8.0. Preferably the denture adhesive composition has (i) a viscosity of from 500,000-2,000,000 centipoise ((cP) measured at 25°C ± 1°C) and/or (ii) a pH of from 4.0 to 10, particularly a pH of from 5.0 to 8.0.

In certain preferred embodiments, the denture adhesive composition comprises or consists essentially of:
(a) from 31-34% w/w of poly(methylvinylether/maleic acid) Sodium-Calcium Mixed Partial Salt;
(b) from 15-23% w/w of sodium carboxymethylcellulose;
(c) from 16-18% w/w of an oil component;
(d) from 28%-30% of petrolatum; and optionally
(e) at least one flavour component, fragrance, colorant or mixture thereof.

In such preferred embodiments the denture adhesive composition has a viscosity of from 800,000 to 2,000,000 centipoise (cP) and a pH of from 5.0 to 8.0.

Particularly when applied to a support surface (carrier) the denture adhesive exhibits work of adhesion values of at least 5 J/m², for up to 20 dislodgement cycles in an Instron based *in vitro* adhesion model.

Particularly, the denture adhesive compositions do not contain polyethylene glycol, sodium alginate and/or zinc. Particularly, denture adhesive compositions of the Invention are free of polyethylene glycol, sodium alginate and zinc.

In a Second Aspect, there is provided a denture adhesive composition according to the First Aspect for use in a method of adhering a dental appliance to a surface, particularly a surface in the oral cavity. There is also provided a method of adhering a dental appliance to a surface, particularly a surface in the oral cavity, using a denture adhesive composition according to the First Aspect. There is also provided a denture adhesive composition according to the First Aspect for use in a method of improving food seal of a dental appliance. There is also provided a denture adhesive composition according to the First Aspect for use in a method of preventing or reducing ingress of food particles between a dental appliance and a surface in the oral cavity. Particularly, the methods may comprise applying a composition of the First Aspect to a dental appliance, the oral cavity, or both, and applying the dental appliance to a surface of the oral cavity, such as the palate or a gum ridge.

### DESCRIPTION OF FIGURES

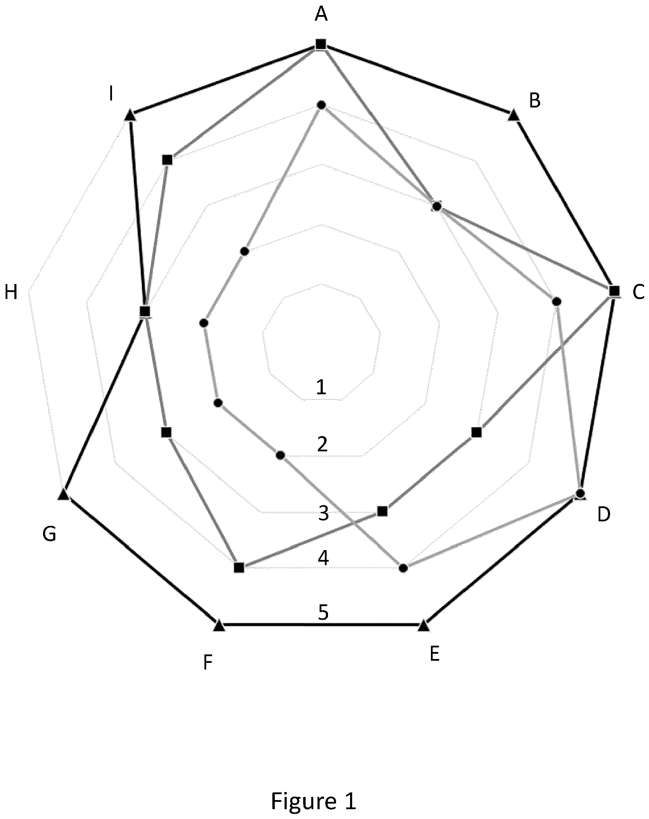

**FIGURE 1****:** Qualitative Patient Testing was performed to compare Poligrip Max Hold & Seal (●), Formulation 1 (▲) and Formulation 6 (■) with 48 partial/ full denture wearers trialing two of the formulations each to qualitatively determine performance. The formulations were scored from 1 to 5 (5 being the best score) in relation to nine sensory attributes, namely: (A) Ease of Application, (B) Appearance Cueing Hold, (C) Initial Mouth Feel, (D) Perceived Hold Throughout Day, (E) Lack of Oozing, (F) Food block/Seal, (G) No Need to Remove and Rinse, (H) Removal, (I) Fit to Product Description.

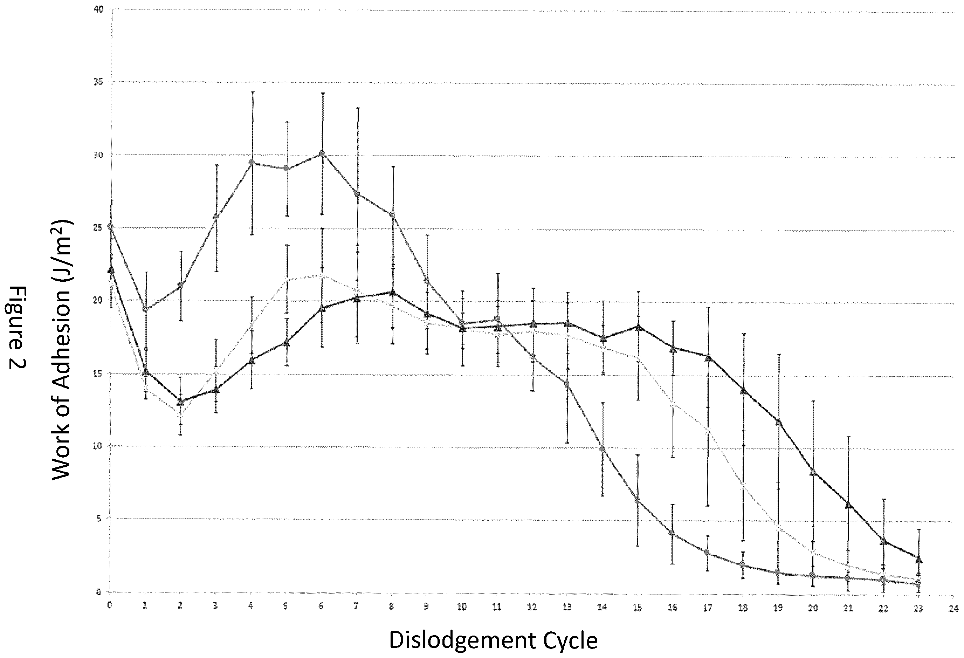

**FIGURE 2****:** Comparison of hold performance (Work of Adhesion) for Formulation 1 (▲) Poligrip Max Hold & Seal (**X**) and Fixodent Professional (●). Formulation 1 demonstrated hold performance comparable with or improved over the existing commercial formulations, exhibiting Work of Adhesion scores above 5J/m² for at least 20 cycles.

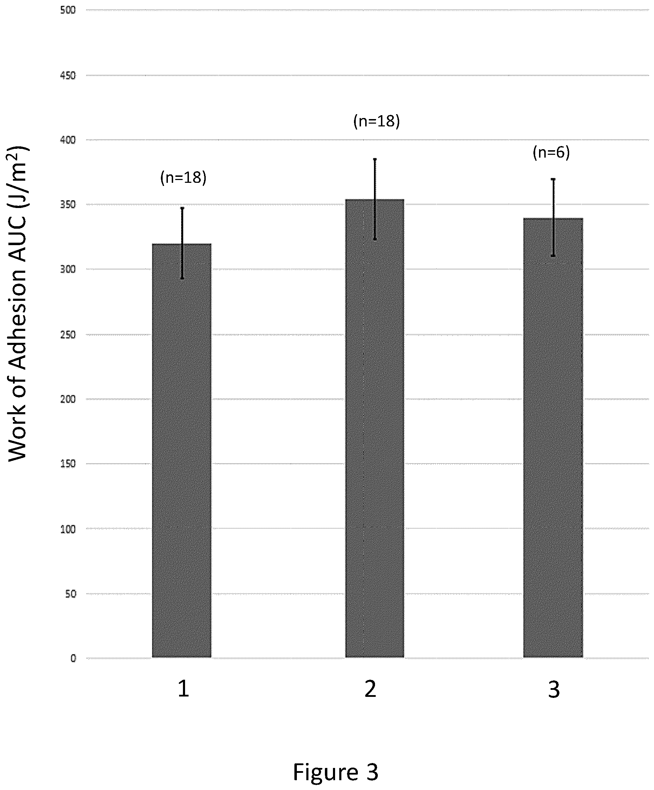

**FIGURE 3****:** Using Instron generated data measured over multiple cycles, the Area Under the Curve (AUC) was calculated for Poligrip Max Hold & Seal **(1),** Formulation 1 (2) and Fixodent Professional (3).

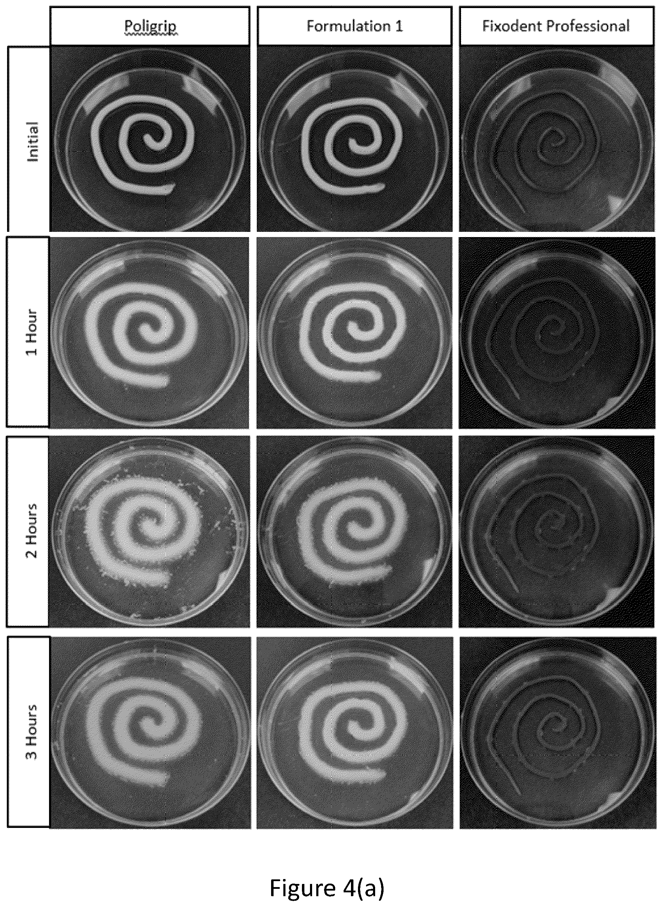

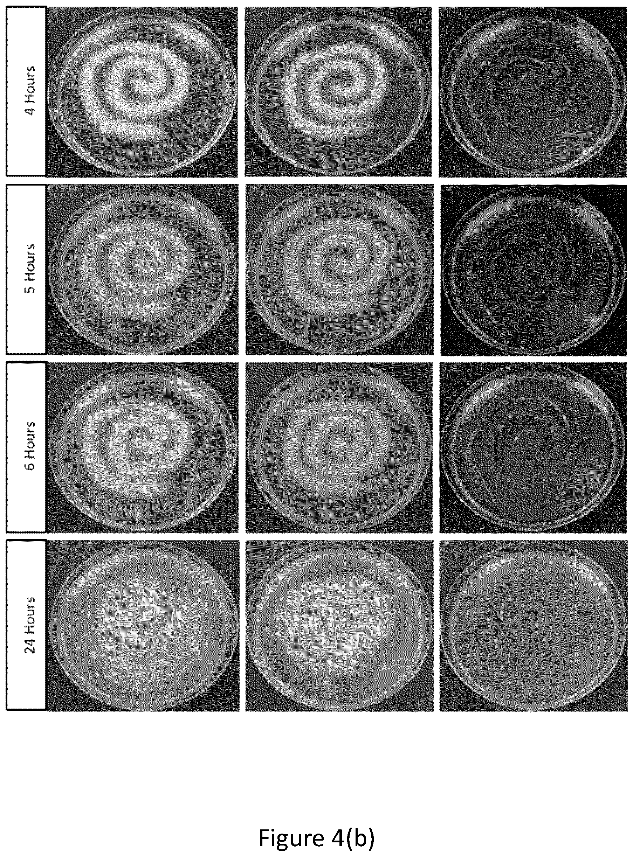

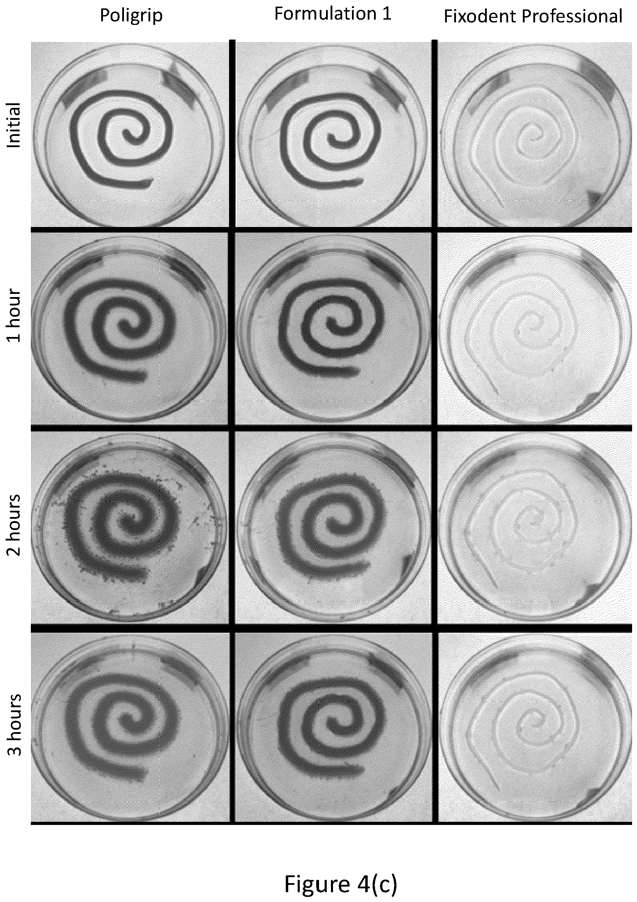

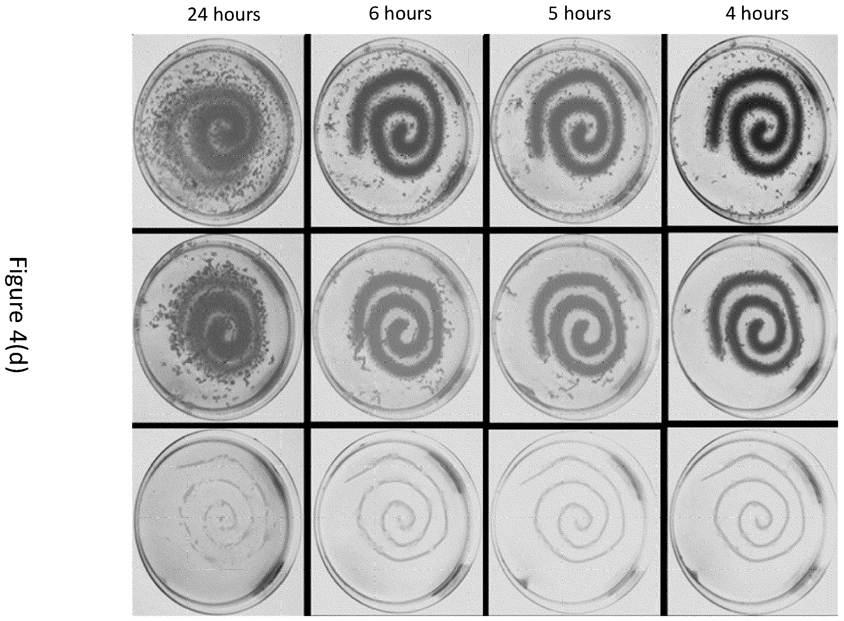

**FIGURE 4****:** Visualisation of swelling (hydration) and disintegration was performed by applying Poligrip Max Hold & Seal, Formulation 1, and Fixodent Professional in a swirling pattern to a petri dish. 40mL of water (40°C) was added to the petri dish slowly via a syringe until the adhesive sample was fully covered. Images were obtained and compared at 0 min, 1 hour, 2 hours, 3 hours, 4 hours, 6 hours and 24 hours (Figure 4(a) and 4(b)). Because Fixodent Professional is a coloured formulation, to aid visualisation these images are reproduced as tonally reversed, negative images in the following Figures 4(c) and (d).

### DETAILED DESCRIPTION OF THE INVENTION

The present inventors have discovered improved denture adhesive compositions that address a number of concerns with currently available denture adhesives, particularly by providing an immediate, strong and long-lasting hold whilst also providing excellent food sealing/blocking properties. In testing, the compositions consistently blocked food ingress throughout the day overcoming the need to remove dentures and re-apply adhesive after eating. In addition, the compositions of the invention exhibit a reduction in disintegration over time, particularly reduced disintegration in water over the course of 24 hours. Not wishing to be bound by theory, this reduction may lead to improvements in consumer perception of the compositions, for example, providing improved cushioning effects and/or reducing the amount of adhesive oozing out of the denture and into the mouth over time.

Denture adhesive compositions of the Invention comprise: (a) from about 31 to about 34 percent by weight (%w/w) of an alkyl vinyl ether-maleic acid copolymer or salt thereof, (b) from about 15 to about 24 percent by weight (%w/w) of carboxymethylcellulose, (c) from about 16 to about 18 percent by weight (%w/w) of an oil component and (d) from about 20 to about 30 percent by weight (%w/w) of petrolatum.

As used herein, the term "dental appliance" refers to dentures or partial dentures, artificial teeth, removable orthodontic bridges and denture plates, both upper and lower types, orthodontic retainers and appliances, protective mouthguards, nightguards to prevent bruxism and/or Temporomandibular joint (TMJ) disorder, and the like. The term "denture" is used to refer to a subset of dental appliances which includes dentures or partial dentures, such as upper and lower dentures and combinations thereof, including orthodontic bridges, artificial teeth, denture plates and the like.

The terms "denture adhesive composition" and "denture adhesive" are terms used in the art and refer to a composition for use with a denture to enhance retention, stability and function.

For the avoidance of doubt, reference to "% by weight" (%w/w) means by weight of the total composition, the amount of each ingredient being selected so that total ingredients in the composition sum to 100 percent by weight.

### Alkyl vinyl ether-maleic acid copolymer

The denture adhesive compositions of the Invention comprise an alkyl vinyl ether-maleic acid (AVE/MA) copolymer or salt thereof, such as a mixed salt, a partial salt or a mixed partial salt. As used herein, the term "mixed salt" refers to salts wherein at least two different cations are mixed on the same polymer with each other or with other salts. The term, "partial salt" refers to salts of polymers where less than 100 percent of the acid groups are reacted.

Particularly, the denture adhesive compositions comprise an alkyl vinyl ether-maleic acid copolymer partial salt. More particularly, the denture adhesive compositions comprise an alkyl vinyl ether-maleic acid copolymer mixed partial salt.

The alkyl vinyl ether-maleic acid copolymer comprises at least one cationic salt selected from the group consisting of strontium, zinc, iron, magnesium, calcium and sodium. Preferably, the alkyl vinyl ether-maleic acid copolymer contains at least one cationic salt selected from the group consisting of calcium and sodium. Thus, in some embodiments the AVE/MA copolymer contains a cationic salt selected from calcium and sodium or a combination thereof. Preferably, the at least one cationic salt is a combination of calcium and sodium cations.

The metal cations may be present on the alkyl vinyl ether-maleic acid copolymer in an amount to provide from about 10.0 percent to about 20.0 percent of metal cations, such as about 12.0% to about 16.0%, for example about 12.0%, about 12.5%, about 13.0%, about 13.5%, about 14.0%, about 14.5%, about 15.0% or about 15.5%.

Particularly, the alkyl vinyl ether-maleic acid copolymer comprises or consists essentially of from about 1.0 percent to about 15.0 percent calcium cations and from about 0.5 percent to about 5.0 percent sodium cations. More particularly, the alkyl vinyl ether-maleic acid copolymer comprises or consists essentially of from about 9.0 percent to about 15.0 percent calcium cations and from about 1.0 percent to about 3.0 percent sodium cations. Preferably the alkyl vinyl ether-maleic acid copolymer is free of zinc cations.

Particularly, the denture adhesive composition comprises from about 31 to about 34 percent by weight of the alkyl vinyl ether-maleic acid copolymer or salt thereof. More particularly, the denture adhesive composition comprises 32 percent by weight of the alkyl vinyl ether-maleic acid copolymer or salt thereof. In other embodiments, the denture adhesive composition comprises about 34 percent by weight of the alkyl vinyl ether-maleic acid copolymer or salt thereof.

Preferably, the alkyl vinyl ether-maleic acid copolymer or salt thereof is a poly(methylvinylether/maleic acid) salt. More preferably, the alkyl vinyl ether-maleic acid copolymer or salt thereof is a poly(methylvinylether/maleic acid) Sodium-Calcium Mixed Partial Salt. Examples of suitable commercially available alkyl vinyl ether-maleic acid copolymers are sold under the trade name GANTREZ (ISP Investments LLC) available from Thermo Fisher or Ashland. These include the the GANTREZ MS form such as GANTREZ MS- 955 (a mixed sodium and calcium salt of methyl vinyl ether and maleic anhydride copolymer).

### Carboxymethylcellulose

Denture adhesive compositions of the Invention comprise carboxymethylcellulose (CMC), a cellulose derivative. Particularly, the compositions comprise from about 15% to about 24% by weight of CMC, particularly from about 20% to about 24% by weight for example, 20%, 21%, 22%, 23% or 24% by weight of CMC, particularly 22% by weight of CMC. Preferably the CMC is food or pharmaceutical grade CMC.

Particularly, the CMC is provided in the form of a powder prior to use in a composition of the Invention, more particularly in the form of a fine powder, more particularly in the form of a fine powder of which at least 99.9% of the particles pass through a 60 U.S. mesh sieve, more particularly at least 90% of the particles pass through an 80 U.S. mesh sieve, yet more particularly at least 80% of the particles pass through a 100 U.S. mesh sieve and still yet more particularly at least 80% of the particles pass through a 200 U.S. mesh sieve

Particularly, the CMC has a viscosity of from about 800 to about 3000 centipoise (cP), preferably a viscosity of from about 1000 to about 2800 cP (measured in a 1% solution at 25°C).

Particularly, the CMC has a degree of substitution (DS) of from about 0.65 to about 0.95, such as from about 0.65 to about 0.85, preferably about 0.7.

Preferably the CMC is sodium carboxymethylcellulose. Particularly the sodium carboxymethylcellulose having a sodium content of from 6.5% to 9.5% such as from 7.0% to about 9.2%, more particularly of from about 7.0% to about 8.9%.

Preferably the CMC is sodium carboxymethylcellulose having an average molecular weight of about 725,000.

Examples of commercially available CMCs useful in compositions of the Invention include the 7H products available from Aqualon such as 7H3SXF or 7H3SF and products available from Dupont such as WALOCEL CRT 2000, WALOCEL CRT 10000 or WALOCEL CRT 15000, such as WALOCEL CRT 15000PPA (S) Sodium. In certain embodiments, the sodium CMC is sodium CMC 7H3SXF, 7H3SF, 7H3SXF PH, 7H3SF PH, WALOCEL CRT 15000, WALOCEL CRT 15000PPA (S) Sodium or equivalents.

In certain embodiments, the sodium carboxymethylcellulose is food or pharmaceutical grade CMC having a viscosity of from about 1000 to about 2800 cP (measured in a 1% solution at 25°C), a degree of substitution of from about 0.65 to about 0.95, a sodium content of from 6.5% to 9.5% and an average molecular weight of about 725,000.

### Oil Component

The oils useful in the compositions of the Invention may have a kinematic viscosity of from about 10 centistokes (cSt, mm²/s) up to about 240 cSt at 40°C, more particularly from about 10 cSt to about 100 cSt at 40°C or yet more particularly from about 10 cSt to about 90 cSt at 40°C. Kinematic viscosity may be measured at 40°C by means of a calibrated glass capillary viscometer as set out in the ASTM Standard Test Method for Kinematic Viscosity of Transparent and Opaque Liquids D445 (DOI: 10.1520/D0445-21E01).

Suitable oils for use in compositions of the Invention include, by way of non-limiting example, light mineral oil, medium mineral oil, vegetable oils such as olive oil, oleic acid, sesame oil, safflower oil, corn oil, soybean oil, cottonseed oil, castor oil, palm oil and coconut oil.

Preferably the oil component is selected from the group consisting of light mineral oil, medium mineral oil and combinations thereof.

Mineral oils may be characterised in terms of their kinematic viscosity. A light mineral oil will generally have a kinematic viscosity of about 34 cSt or less at 40°C. Medium and heavy mineral oils will generally have a kinematic viscosity ranging from about 35 cSt up to about 240 cSt at 40°C.

In certain embodiments, the oil component is medium mineral oil. In certain embodiments, the oil is a medium mineral oil, more particularly a medium mineral oil having a kinematic velocity of from about 35 cSt to about 90 cSt at 40°C and yet more particularly a medium mineral oil having a kinematic velocity of from about 63 cSt to about 80 cSt at 40°C. Suitable medium mineral oils include PIONIER 2071P or equivalent.

In certain embodiments, the oil component is light mineral oil. In certain embodiments, the oil is a light mineral oil, more particularly a light mineral oil having a kinematic velocity of from about 10 cSt to about 34 cSt at 40°C, yet more particularly a medium mineral oil having a kinematic velocity of from about 10 cSt to about 20 cSt at 40°C, still yet more particularly a medium mineral oil having a kinematic velocity of from about 14 cSt to about 17 cSt at 40°C. Suitable light mineral oils include BLANDOL white mineral oil or equivalent.

Particularly, the compositions comprise from about 16% to about 18% by weight (%w/w) of an oil component, for example, 16%, 17% or 18% by weight of an oil component, particularly 17% by weight of an oil component. Light mineral oils (as defined above) are preferred for use in the Invention. Thus, the compositions comprise from about 16% to about 18% by weight (%w/w) of light mineral oil, for example, 16%, 17% or 18% by weight of light mineral oil, particularly 17% by weight of light mineral oil.

### Petrolatum

The term "petrolatum" is a term of the art used to refer to a semi-solid mixture of hydrocarbons. The viscosity of petrolatum may be determined using the ASTM Standard Test Method for Cone Penetration of Petrolatum (D937-07) to determine the penetration of petrolatum as an empirical measure of consistency. The petrolatum may have a cone penetration consistency value (mm/10 at 25°C) of from about 10 to about 500, particularly from about 25 to about 300 and more particularly from about 50 to about 250. Preferably, the petrolatum has a cone penetration consistency value of from about 100 to about 200, more preferably from about 130 to about 195 (mm/10 at 25°C).

Particularly, denture adhesive compositions of the Invention may comprise from about 20 to about 30 percent by weight (%w/w) of petrolatum, more particularly from about 25 to about 30 percent by weight (%w/w) of petrolatum, for example 25%, 26%, 27%, 28% or 29% by weight (%w/w) of petrolatum. Yet more particularly, denture adhesive compositions of the Invention comprise about 29 percent by weight (%w/w) of petrolatum.

### Other Components

Denture adhesive compositions may comprise at least one sensory component. A "sensory component" refers to a component that may be used to impart a flavour, fragrance, colour and/or sensation (such as cooling, warming or tingling) to the composition. Other than imparting the compositions with organoleptic properties that are desirable for consumers, generally the sensory component is not an active component, in other words it does not play an active role in adhesion, hold time or cushioning. Particularly, the sensory component is at least one flavour, fragrance and/or colouring agent. Sensory components are known in the art and may be natural or artificial (synthetic).

Compositions of the invention may comprise from about 0.01% to about 5% by weight of a sensory component, particularly from about 0.01% to about 2% by weight of a sensory component.

Flavouring agents well known in the denture adhesive art may be added to the compositions of the present invention. These flavouring agents include, but are not limited to, synthetic flavour oils and/or oils derived from plants, leaves, flowers, fruits and so forth, and combinations thereof. Representative flavour oils include spearmint oil, cinnamon oil, oil of wintergreen (methylsalicylate) and peppermint oils. Also useful are artificial, natural or synthetic fruit flavours such as citrus oil including lemon, orange, grape, lime, and grapefruit, and fruit essences including apple, strawberry, cherry, pineapple, and so forth. The flavouring agent may be a liquid, spray dried, encapsulated, or absorbed on a carrier, and mixtures thereof. In one embodiment, the flavouring agent is peppermint oil. The amount of flavouring agent utilized varies depending on such factors as flavour type, adhesive composition and strength desired. In certain embodiments, the denture adhesive composition comprises from about 0.01 percent to about 3.0 percent by weight of the flavouring agent. In certain embodiments, the denture adhesive composition comprises from about 0.01 percent to about 2.0 percent by weight of the flavouring agent. In certain embodiments, the denture adhesive composition comprises from about 0.05 percent to 2.0 percent by weight of the flavouring agent.

The denture adhesive compositions may also include the use of sweeteners well known in the art. The sweetening agent may be selected from a wide range of materials including water-soluble agents, water-soluble artificial sweeteners, and dipeptide-based sweeteners, including mixtures thereof. Representative sweeteners include, but are not limited to, (a) sugar alcohols such as sorbitol, xylitol, mannitol, maltitol, hydrogenated starch hydrolysate, and mixtures thereof; (b) water-soluble artificial sweeteners such as the soluble saccharin salts, i.e., sodium or calcium saccharin salts, cyclamate salts, Acesulfame-K, sucralose, and the like, and the free acid form of saccharin; (c) dipeptide based sweeteners such as L-aspartyl-L-phenylalanine methyl ester, and the like; and (d) natural sweeteners such as stevia. In certain embodiments, the denture adhesive composition comprises from about 0.001 percent to about 5.0 percent by weight of the sweetening agent.

The colourants useful in the present invention include the pigments and dyes suitable for food, drug and cosmetic applications. These colourants are known as FD&C (Food, Drugs and Cosmetics) and D&C (Drugs & Cosmetics) dyes and lakes. Illustrative examples include, but are not limited to, D&C red#30 Aluminium Lake Paste, D&C Red #7 Calcium Lake Paste, Erythrosine Lake Paste, indigo dye, known as FD&C Blue No. 2, which is the disodium salt of 5,5 -indigotindi-sulfonic acid; FD&C Green No. 1, comprising a triphenylmethylene dye and is the monosodium salt of the 4-[4-N- ethyl-p-sulfobenzylamino) diphenylmethylene]-[I-(N-ethyl-N-P-sulfobenzyl)-2,5- cyclohexadienimine]. In some embodiments, the colorant is FD&C Red No. 3. In some embodiments, the denture adhesive composition comprises from about 0 percent to about 0.2 percent by weight of colourant.

In certain embodiments, the denture adhesive composition is free of flavour, fragrance and/or colouring agents, i.e. it does not comprise flavour, fragrance and/or colouring agents (beyond the intrinsic properties of components (a), (b) (c) and (d).

Particularly when applied to a support surface (carrier) the denture adhesive exhibits work of adhesion values of 5 J/m² or higher, for at least 20 dislodgement cycles in an Instron based *in vitro* adhesion model.

Denture adhesive compositions of the Invention may be dispensed by the consumer from a multidose package, pump or tube. Alternatively, the denture adhesive compositions of the Invention may be pre-dispensed, in single dose packages, for example on a non-adhesive self-supporting layer that may be peeled off to enable the adhesive to be applied.

To facilitate dispensing, when measured following preparation of the composition, the denture adhesive compositions of the Invention may have a viscosity of from 500,000-2,500,000 centipoise (cP).

Particularly, following preparation, the denture adhesive compositions of the Invention have a viscosity of at least 500,000 centipoise (cP) or more particularly at least 800,000 centipoise (cP). Particularly, following preparation, the denture adhesive compositions of the Invention have a viscosity of no more than 2,500,000 cP, more particularly no more than 2,000,000 cP. Particularly, following preparation, the denture adhesive compositions of the Invention have a viscosity of from 500,000 to 2,500,000 centipoise (cP), more particularly of from 500,000 to 2,000,000 cP, yet more particularly of from 800,000 to 2,000,000 cP. For the avoidance of doubt, viscosity may be measured within two months of preparation of the composition, more particularly within one month of preparation of the composition, such as a viscosity of from 800,000 to 2,500,000 cP when measured within one month of preparation of the composition. Preferably, viscosity is measured at 25°C ± 1°C.

Compositions of the invention preferably have a pH within the range of from 4.0 to 10.0, such as a pH within the range of from 5.0 to 8.0, preferably a pH of from about 5.0 to about 7.5, such as a pH of from about 5.2 to about 7.1 or a pH of from 6.5 to 7.5.

Particularly the denture adhesive compositions of the Invention have (i) a viscosity of from 500,000 to 2,000,000 centipoise (cP) and/or (ii) a pH of from 4.0 to 10, more particularly a pH of from 5.0 to 8.0. Preferably the denture adhesive composition has (i) a viscosity of from 500,000-2,000,000 centipoise ((cP) measured at 25°C ± 1°C) and/or (ii) a pH of from 4.0 to 10, particularly a pH of from 5.0 to 8.0. In certain embodiments, the denture adhesive composition has (i) a viscosity of from 500,000-2,000,000 centipoise, particularly 800,000-2,000,000, ((cP) measured at 25°C ± 1°C) and (ii) a pH of from 4.0 to 10, particularly a pH of from 5.0 to 8.0.

Typically, compositions of the invention are stable for at least 3 months at a temperature of 25°C, particularly for at least 6 months at a temperature of 25°C, more particularly for at least 12 months at a temperature of 25°C, yet more particularly for at least 24 months at a temperature of 25°C and still yet more particularly for 36 months at a temperature of 25°C. As used herein, the term "stable" refers to the ability of the composition to maintain certain physical or chemical properties, such as adhesion, at a specific storage temperature for a period of time after production. The stability of compositions of the present invention may be assessed using standard methods known in the art.

Particularly, denture adhesive compositions do not contain polyethylene glycol, sodium alginate and/or zinc. Preferably, denture adhesive compositions of the Invention are free of polyethylene glycol, sodium alginate and zinc. Particularly, denture adhesive compositions of the Invention do not comprise di-basic sodium phosphate, tetra sodium pyro phosphate and sodium tri poly phosphate. In certain embodiments of the Invention, the denture adhesive composition consists essentially of: (a) from about 31 to about 34 percent by weight (%w/w) of an alkyl vinyl ether-maleic acid copolymer or salt thereof, (b) from about 15 to about 23 percent by weight (%w/w) of carboxymethylcellulose, (c) from about 16 to about 18 percent by weight (%w/w) of an oil component and (d) from about 20 to about 30 percent by weight (%w/w) of petrolatum. In certain embodiments of the Invention, the denture adhesive composition consists of: (a) from about 31 to about 34 percent by weight (%w/w) of an alkyl vinyl ether-maleic acid copolymer or salt thereof, (b) from about 15 to about 23 percent by weight (%w/w) of carboxymethylcellulose, (c) from about 16 to about 18 percent by weight (%w/w) of an oil component and (d) from about 20 to about 30 percent by weight (%w/w) of petrolatum.

There is also provided a denture adhesive composition of the Invention for use in a method of adhering a dental appliance to a surface, particularly a surface in the oral cavity. There is also provided a method of adhering a dental appliance to a surface, particularly a surface in the oral cavity, using a denture adhesive composition of the Invention. There is also provided a denture adhesive composition of the Invention for use in a method of improving food seal of a dental appliance. There is also provided a denture adhesive composition of the Invention for use in a method of preventing or reducing ingress of food particles from a cavity, particularly the oral cavity of a subject, into the interstice between a dental appliance and the palate or gum ridge of the subject.

Particularly, the methods may comprise applying a composition of the First Aspect to a dental appliance, the oral cavity, or both, and applying the dental appliance to a surface of the oral cavity, such as the palate or a gum ridge.

There is also provided a method of improving, for example increasing, food seal of a dental appliance comprising applying a composition of the Invention to a dental appliance, the oral cavity, or both, and applying the dental appliance to a surface of the oral cavity, such as the palate or a gum ridge.

There is also provided a method of preventing or reducing ingress of food particles from a cavity, particularly the oral cavity of a subject, into the interstice between a dental appliance and the palate or gum ridge of the subject comprising applying a composition of the Invention to a dental appliance, the oral cavity, or both, and applying the dental appliance to a surface of the oral cavity of the subject, such as the palate or a gum ridge.

Food seal refers to providing a seal along the edges of a dental appliance to reduce or prevent ingress of food particles, thereby avoiding food becoming trapped between the dental appliance and the palate or gum ridge where it can cause irritation to the dental appliance wearer. Reference to improving food seal of a dental appliance includes preventing, delaying, and/or reducing the ability of food particles to enter the spaces between a dental appliance and the palate or gum ridge. Food seal or block may be assessed using tests known in the art, more particularly food-occlusion testing performed by assessing peanut particle migration under dentures. Thus, improvements in food seal may be assessed using food-occlusion testing comparing particle migration under dentures with and without the use of denture adhesive. Particularly, using quantitative testing, the use of the compositions of the Invention result in a reduction in the mass of peanut particles found under the denture. Improvements in food seal may also be assessed by qualitative testing in patient trials with and without denture adhesive where participants are asked to rate the quantity of particles they perceive as being under their dentures. Particularly, the use of the compositions of the Invention result in a reduction in the number of participants aware of food particles under their dentures.

### General

The term "comprising" encompasses "including" e.g. a composition "comprising" X may include something additional e.g. X + Y. In some implementations, the term "comprising" refers to the inclusion of the indicated active agent, such as recited compounds, as well as inclusion of other active agents, and carriers, excipients, emollients, stabilizers, etc., known in the consumer health industry and generally recognized as safe (GRAS). Use of the transitional phrase "consisting essentially of" means that the scope of a claim is to be interpreted to encompass the specified materials or steps recited in the claim, and those that do not materially affect the basic and novel characteristic(s) of the claimed invention. See, In re Herz, 537 F.2d 549, 551-52, 190 USPQ 461, 463 (CCPA 1976) (emphasis in the original); see also MPEP § 2111.03. Thus, the term "consisting essentially of" when used in a claim of this invention is not intended to be interpreted to be equivalent to "comprising". The term "consisting of" and variations thereof means including and limited to (for example, the specific recited constituents or steps). In certain territories, the term "comprising an active ingredient consisting of" may be used in place of "consisting essentially". The term "about" in relation to a numerical value x is optional and means, for example, that the numerical value may comprise some variance around the stated number to allow for routine experimental fluctuation, measurement variance or to encompass minor deviations that may achieve substantially the same results as the stated number, such as x±10%, x±5%, x±4%, x±3%, x±2% or x±1%. The word "substantially" does not exclude "completely" e.g. a composition which is "substantially free" from Y may be completely free from Y. Where necessary, the word "substantially" may be omitted from the definition of the invention. All percentages and ratios used herein are by weight of total composition, unless otherwise indicated.

All references or patent applications cited within this patent specification are incorporated by reference herein.

### Embodiments of the Invention

The following clauses describe certain specific embodiments of the invention:
**EMBODIMENT 1:** A denture adhesive composition comprising or consisting essentially of:
   (a) from 31-34% w/w of poly(methylvinylether/maleic acid) Sodium-Calcium Mixed Partial Salt;
   (b) from 15-24% w/w of sodium carboxymethylcellulose;
   (c) from 16-18% w/w of an oil component;
   (d) from 28%-30% of petrolatum; and optionally
   (e) at least one sensory component, for example a flavour component, fragrance, colorant or mixture thereof.
**EMBODIMENT 2:** The denture adhesive composition of EMBODIMENT 1 having a viscosity of from 800,000 to 2,000,000 centipoise (cP) and a pH of from 5.0 to 7.5.
**EMBODIMENT 3:** A denture adhesive composition consisting essentially of:
   (a) from 31-34% w/w of poly(methylvinylether/maleic acid) Sodium-Calcium Mixed Partial Salt;
   (b) from 15-24% w/w of sodium carboxymethylcellulose;
   (c) from 16-18% w/w of an oil component;
   (d) from 28%-30% of petrolatum; wherein
   the denture adhesive composition has a viscosity of from 800,000 to 2,000,000 centipoise (cP) and a pH of from 5.0 to 7.5, particularly a pH of from 6.0 to 7.5.
**EMBODIMENT 4:** A denture adhesive composition comprising or consisting essentially of (a) 32% w/w of poly(methylvinylether/maleic acid) Sodium-Calcium Mixed Partial Salt; (b) 22% w/w of sodium carboxymethylcellulose; (c) 17% w/w of an oil component; (d) 29% of petrolatum; and optionally (e) at least one sensory component, for example a flavour component, fragrance, colorant or mixture thereof.
**EMBODIMENT 5:** The denture adhesive composition of EMBODIMENT 4 having a viscosity of from 800,000 to 2,000,000 centipoise (cP) and a pH of from 5.0 to 7.5, particularly a pH of from 6.0 to 7.5.
**EMBDOIMENT 6:** The denture adhesive composition of EMBODIMENT 4 or 5, wherein the oil component is light mineral oil.
**EMBODIMENT 7:** A denture adhesive composition consisting essentially of (a) 32% w/w of poly(methylvinylether/maleic acid) Sodium-Calcium Mixed Partial Salt; (b) 22% w/w of sodium carboxymethylcellulose; (c) 17% w/w of light mineral oil; (d) 29% of petrolatum; wherein the denture adhesive composition has a viscosity of from 800,000 to 2,000,000 centipoise (cP) and a pH of from 5.0 to 7.5, particularly a pH of from 6.0 to 7.5.
**EMBODIMENT 8:** A denture adhesive composition comprising or consisting essentially of (a) 34% w/w of poly(methylvinylether/maleic acid) Sodium-Calcium Mixed Partial Salt; (b) 24% w/w of sodium carboxymethylcellulose; (c) 17% w/w of an oil component; (d) 25% of petrolatum; and optionally (e) at least one flavour component, fragrance, colorant or mixture thereof.
**EMBODIMENT 9:** The denture adhesive composition of EMBODIMENT 8 having a viscosity of from 800,000 to 2,000,000 centipoise (cP) and a pH of from 5.0 to 7.5, particularly a pH of from 6.0 to 7.5.
**EMBODIMENT 10:** A denture adhesive composition consisting essentially of (a) 34% w/w of poly(methylvinylether/maleic acid) Sodium-Calcium Mixed Partial Salt; (b) 24% w/w of sodium carboxymethylcellulose; (c) 17% w/w of medium viscosity mineral oil; (d) 25% of petrolatum; wherein the denture adhesive composition has a viscosity of from 800,000 to 2,000,000 centipoise (cP) and a pH of from 5.0 to 7.5, particularly a pH of from 6.0 to 7.5.

In order that this invention may be better understood, the following examples are set forth. These examples are for purposes of illustration only and are not to be construed as limiting the scope of the invention in any manner.

### EXAMPLES

### Example 1: Denture adhesive compositions I

Five formulations were prepared using commercially available ingredients with the aim of providing better hold (adhesion over time) whilst also providing improvements in consumer sensorial experience such as, ease of application, feel across and throughout the day, ease of removal:

| **Raw Material** | **1** | **2** | **3** | **4** | **5** |
|---|---|---|---|---|---|
| | +2% pol (-CMC) | +4% pol (-CMC) | +2% pol (-Pet) | +4% pol (-Pet) | +3% CMC (-pet) |
| Poly(Methylvinylether/maleic acid) Sodium-Calcium Mixed Partial Salt (Polisalt) | 32.00 | 34.00 | 32.00 | 34.00 | 30.00 |
| Petrolatum Blend | 29.00 | 29.00 | 27.00 | 25.00 | 26.00 |
| Carboxymethyl Cellulose (CMC) | 22.00 | 20.00 | 24.00 | 24.00 | 27.00 |
| Liqht Mineral Oil | 17.00 | 17.00 | 17.00 | 17.00 | 17.00 |
| Total | 100.00 | 100.00 | 100.00 | 100.00 | 100.00 |

Initial physical test results of the five test formulations indicated formula 1 to be most promising due to viscosity and cost parameters comparable with commercially available formulations.

| | **1** | **2** | **3** | **4** | **5** |
|---|---|---|---|---|---|
| Appearance | Homogeneous, smooth paste | Homogeneous, smooth paste | Homogeneous, smooth paste | Homogeneous, smooth paste | Homogeneous, smooth paste |
| Extrudability | Easy | Easy | Easy | Easy | Easy |
| Viscosity (cPs) | 1,028,800 | 962,800 | 1,370,000 | 1,374,000 | 1,678,000 |
| ISO Adhesive Strength Test (Kpa) | 50.17 | 52.24 | 49.704 | 45.826 | 48.599 |
| Shear strength (Lbf) | 3.444 | 2.632 | 3.112 | 3.233 | 2.885 |
| pH (ISO Test Method) | 6.955 | 7.018 | 7.214 | 7.143 | 7.076 |
| **Cost** | 5.91 | 6.14 | 5.94 | 6.19 | 5.73 |
| **Notes** | Viscosity and cost acceptable | Higher cost | higher viscosity | Higher viscosity and higher cost | Viscosity too high |

### Example 2: Denture adhesive compositions II

Three further formulations were generated and compared with formulation 1. Formulation 8 comprised a softer petrolatum (Pionier 5741) with the aim of reducing the viscosity of the formulation that was predicted to be increased as a result of the polisalt content:

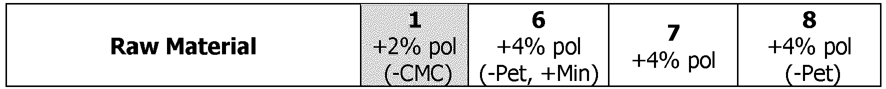

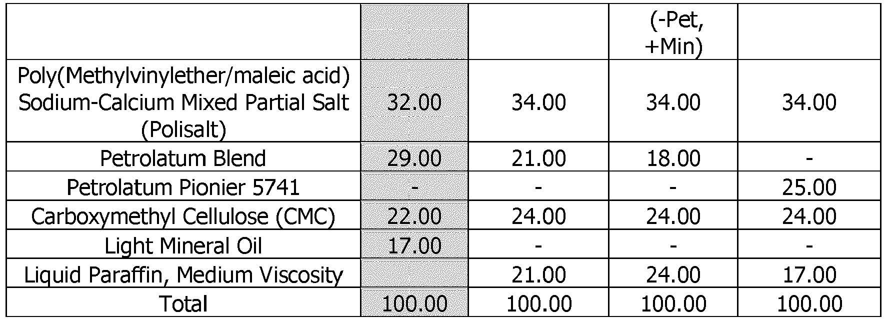

The formulations were tested, as before, and Formulations 1 and 6 were selected for patient testing based on *in vitro* performance:

| | **1** | **6** | **7** | **8** |
|---|---|---|---|---|
| **Appearance** | Homogeneous, smooth to slightly grainy paste and No syneresis | Homogeneous, smooth to slightly grainy off- white paste with no syneresis | Homogeneous, smooth to slightly grainy off- white paste with no syneresis | Homogeneous, smooth to slightly grainy off- white paste with no syneresis |
| **Extrudability** | Easy | Easy | Easy | Easy |
| **Syneresis** | No | No | No | No |
| **Viscosity (cPs)-Initial** | 977,333 | 1,426,000 | 1,162,800 | 914,000 |
| **Adhesive Strength - 10 mins (KPa)** | 49.921 | 45.94 | TBU | TBU |
| **Shear Strength (lbf)** | 3.147 | 2.854 | 2.935 | 2.776 |
| **pH (ISO Test method)** | 6.955 | 7.127 | 7.014 | 7.079 |
| **Work of Adhesion Area Under Curve (J/m^2)** | 354 | 438 | 417 | 372 |
| **Notes** | Low viscosity and higher adhesion than control. | Viscosity too high, difficult to squeeze, best adhesion profile | High adhesion and good viscosity but somewhat difficult to squeeze | Low viscosity and higher adhesion than control |

### Example 3: Qualitative Patient Testing

Using Poligrip Max Hold & Seal (a commercially marketed denture adhesive) as a control, Formulations 1 and 6 were tested with 48 partial/ full denture wearers with all patients trialing two of the formulations to qualitatively determine performed best in relation to nine sensory attributes, namely: Ease of Application, Appearance, Initial Mouth Feel, Perceived Hold, Lack of Oozing, Food block/Seal, Need to Remove and Rinse, Removal, Fit to Product Description. Formulation 1 exceeded the performance of Formulation 6 in eight out of nine qualitative attributes and the control in seven out of nine qualitative attributes (Figure 1).

### Example 4: Work of Adhesion

To compare the adhesion profile of Formulation 1 over time with two commercially available denture adhesives (Fixodent Professional and Poligrip Max Hold & Seal), an Instron Texture Analyzer (5943) equipped with automated spray and 500 N load cell was used (Instron, UK). A quantity of adhesive was placed onto the test surface of the texture analyser, compressed to a uniform thickness of 1mm across the test surface and then taken through a number of cycles of compression/stretching. Following the compression/stretching cycles, the cross head was rapidly moved apart with speed of 6lbf/min and the maximum tensile force recorded. This was repeated at least 20 times for each sample.

Formulation 1 demonstrated hold performance comparable with or improved over the Poligrip Max Hold & Seal and Fixodent Professional (Figure 2). Indeed, Formulation 1 exhibited Work of Adhesion scores above 5J/m² for at least 20 cycles.

Fixodent Professional exhibited sub-optimal performance in comparison to both Formulation 1 and Poligrip Max Hold & Seal.

With regard to Area under Curve (Figure 3) Formulation 1 demonstrated directionally better hold than both commercial formulations.

### Example 5: Swelling/Disintearation Testing

Visual analysis of swelling (hydration) and disintegration over time was performed by applying Formulation 1, Poligrip Max Hold & Seal and Fixodent Professional in a swirling pattern to a petri dish. 40mL of water (40°C) was added to the petri dish slowly via a syringe until the adhesive sample was fully covered. Images were obtained at 0 min, 1 hours, 2 hours, 3 hours, 4 hours, 6 hours and 24 hours (Figures 4(a) and 4(b)). Because Fixodent Professional is a coloured formulation, the images were also tonally reversed as negative images to aid visualization (Figures 4(c) and 4(d)).

Visual results indicated that Formulation 1 did not disintegrate as much as the two commercial formulations under the same conditions (40mL water at 40°C at specific time points). Formulation 1 also appeared to be less soluble than the two commercial formulations indicative that it would remain intact for longer in the oral cavity.

Overall, Formulation 1 exhibited improved hold and sensorial attributes outperforming a number of other formulations.

The purpose of the above description is to illustrate some embodiments of the present invention without implying a limitation. It will be apparent to those skilled in the art that various modifications and variations may be made in the apparatus or procedure of the invention without departing from the scope or spirit of the invention.

## Claims

1. A denture adhesive composition comprising: (a) from about 31 to about 34 percent by weight (%w/w) of an alkyl vinyl ether-maleic acid copolymer or salt thereof, (b) from about 15 to about 24 percent by weight (%w/w) of carboxymethylcellulose, (c) from about 16 to about 18 percent by weight (%w/w) of the oil component and (d) from about 20 to about 30 percent by weight (%w/w) of petrolatum.

2. The denture adhesive composition of Claim 1, wherein the alkyl vinyl ether-maleic acid copolymer or salt thereof is a poly(methylvinylether/maleic acid) salt.

3. The denture adhesive composition of Claim 2, wherein the alkyl vinyl ether-maleic copolymer or salt thereof is a poly(methylvinylether/maleic acid) Sodium-Calcium Mixed Partial Salt.

4. The denture adhesive composition of Claim 3, wherein the carboxymethylcellulose has a viscosity of about 1000 to about 2800 centipoise (cP) (measured in a 1% solution at 25°C).

5. The denture adhesive composition of Claim 5, wherein the carboxymethylcellulose consists of fine particles of which at least 80% pass through a 200 U.S. mesh sieve (equivalent to a 74 µm mesh sieve).

6. The denture adhesive composition of any one of Claims 4 or 5, wherein the carboxymethylcellulose is sodium carboxymethylcellulose optionally having a degree of substitution (DS) of from 0.65 to 0.95, particularly about 0.7.

7. The denture adhesive of Claim 6, wherein the oil component is light mineral oil.

8. The denture adhesive of Claim 6 or 7, wherein the petrolatum has a cone penetration consistency value of from about 100 to about 200 (mm/10 at 25°C).

9. The denture adhesive of Claim 8, wherein the petrolatum has a cone penetration consistency value of from about 130 to about 195 (mm/10 at 25°C).

10. The denture adhesive composition of any one of Claims 6 to 9, wherein the composition has (i) a viscosity of from 500,000-2,000,000 centipoise ((cP) measured at 25°C ± 1°C) and/or (ii) a pH of from 4.0 to 10, particularly a pH of from 5.0 to 8.0.

11. The denture adhesive composition of any preceding claim which comprises or consists essentially of:
(a) from 31-34% w/w of poly(methylvinylether/maleic acid) Sodium-Calcium Mixed Partial Salt;
(b) from 15-24% w/w of sodium carboxymethylcellulose;
(c) from 16-18% w/w of an oil component;
(d) from 28%-30% of petrolatum; and optionally
(e) at least one flavour component, fragrance, colorant or mixture thereof.

12. The denture adhesive composition of Claim 11, wherein the composition has a viscosity of from 800,000 to 2,000,000 centipoise (cP) and a pH of from 5.0 to 7.5.

13. The denture adhesive of Claim 11 or 12, wherein when applied to a support surface (carrier) the denture adhesive exhibits work of adhesion values of at least 5 J/m², for up to 20 dislodgement cycles in an Instron based in vitro adhesion model.

14. The denture adhesive composition of any one of Claims 1 to 13 wherein the composition is free of polyethylene glycol, sodium alginate and zinc.
